# EUROPÄISCHE PATENTSCHRIFT

(11) **EP 0 954 246 B1**
(45) Veröffentlichungstag und Bekanntmachung des Hinweises auf die Patenterteilung: **22.03.2006**
(21) Anmeldenummer: 97952919.5
(22) Anmeldetag: 11.12.1997
(51) Int. Cl.: A61B 18/00

(54) **KOAGULATIONSVORRICHTUNG ZUR KOAGULATION BIOLOGISCHER GEWEBE**
COAGULATION DEVICE FOR COAGULATING BIOLOGICAL TISSUES
DISPOSITIF POUR LA COAGULATION DE TISSUS BIOLOGIQUES

(30) Priorität: 12.12.1996 DE 19651753; 19.12.1996 DE 19653214
(43) Veröffentlichungstag der Anmeldung: 10.11.1999
(73) Patentinhaber: Erbe Elektromedizin GmbH, 72072 Tübingen (DE)
(72) Erfinder: FARIN, Günter, D-72070 Tübingen (DE); GRUND, Karl, Ernst, D-72070 Tübingen (DE)
(74) Vertreter: Bohnenberger, Johannes
(86) Internationale Anmeldenummer: PCT/EP1997/006937
(87) Internationale Veröffentlichungsnummer: WO 1998/025530

(56) Entgegenhaltungen:
- DE-A- 4 139 029
- US-A- 5 207 675

## Beschreibung

Die Erfindung betrifft eine Koagulationsvorrichtung, insbesondere eine Plasma-Koagulationsvorrichtung nach dem Oberbegriff des Patentanspruches 1.

Aus der DE 37 10 489 C2 ist eine Plasma-Koagulationsvorrichtung bekannt, die für die offene Chirurgie gedacht ist. Bei dieser Vorrichtung wird Argon mit einem so hohen Druck und einer so hohen Strömungsgeschwindigkeit aus einer Düse, in deren Zentrum sich eine Entladungselektrode befindet, ausgestoßen, daß zusätzlich zum Koagulationseffekt durch einen Lichtbogen, der sich zwischen der Elektrode und dem zu koagulierenden Gewebe bildet, auf dem Gewebe "schwimmende" Flüssigkeiten verdrängt werden. Bei dieser Anordnung ist die Emboliegefahr sehr groß. Weiterhin ist eine Verwendung dieser Vorrichtung in Körperhöhlen äußerst problematisch, da die zugeführten hohen Gasmengen zu unerwünschten Insufflationseffekten führen können.

Eine Koagulationsvorrichtung für die Anwendung in einem Endoskop ist aus der US 5,207,675 bekannt. Bei dieser Vorrichtung wird ein Schlauch mit darin verschiebbar angeordneter Elektrode so in den Arbeitskanal eines Endoskops eingesetzt, daß er mittels eines Handgriffes wie ein (übliches) Instrument innerhalb des Arbeitskanals manipulierbar ist. Die Elektrode, die gleichzeitig auch als Instrument ausgebildet sein soll, wird im Koagulations-Betriebsmodus, bei welchem Argon aus dem die Elektrode bergenden Schlauch ausgestoßen wird, in einem aus dem Schlauch hervorragenden Zustand gehalten. Wenn in diesem Zustand die Elektrode das zu koagulierende Gewebe berührt, kann es zu erheblichen Gewebeschädigungen kommen. Bei dünnwandigem Gewebe können die Folgen (Durchbruch etc.) fatal sein.

In der DE 195 35 811 A1 ist eine Koagulationsvorrichtung der eingangs genannten Art beschrieben, welche bereits die wesentlichen Probleme der zuvor beschriebenen Anordnungen vermeiden hilft. Insbesondere ist das Ende des die Elektrode bergenden Schlauches so ausgebildet, dass auch bei stärkerer Gaszufuhr keine Emboliegefahr besteht, wobei die Elektrode gleichzeitig so weit in den Gaszufuhrschlauch zurückgezogen angeordnet ist, dass ein direkter Kontakt mit dem Gewebe nicht geschehen kann. Diese Anordnung ist jedoch relativ aufwendig in der Herstellung.

Aus der DE-A-4139029 ist eine Koagulationsvorrichtung nach dem Oberbegriff des Patentanspruches 1 bekannt. Bei dieser wird zwar der Arbeitskanal des Endoskopes als Leitung zum Transport des Edelgases benutzt, jedoch wird auf das distale Ende des Arbeitskanals eine Düse aufgesetzt, innerhalb derer sich die Elektrode befindet. Die sich so ergebende Vorrichtung unterscheidet sich funktionell nicht von den oben beschriebenen Koagulationsvorrichtungen mit den dort beschriebenen Nachteilen.

Der Erfindung liegt die Aufgabe zugrunde, eine Koagulationsvorrichtung aufzuzeigen, die bei verringertem Herstellungsaufwand eine erhöhte Verwendungssicherheit und verbesserte Verwendbarkeit sicherstellt.

Diese Aufgabe wird durch eine Koagulationsvorrichtung nach Anspruch 1 gelöst.

Ein wesentlicher Punkt der Erfindung liegt darin, dass vollständig von der Idee abgerückt wird, das (Edel-)Gas müsse gezielt der Stelle zugeführt werden, an welcher die Koagulation stattfindet, also mittels eines Schlauches, in welchem die Stromzuführungselektrode angeordnet ist. Stattdessen wird beim Erfindungsgegenstand der Arbeitskanal selbst als Leitung benutzt, um das Gas zu transportieren während die Elektrode als vollisolierter Draht ohne Lumen ausgebildet sein kann. Überraschenderweise hat es sich gezeigt, dass keine nachteiligen Effekte dadurch auftreten, dass das Gas ein ganzes Stück vor dem distalen Ende des Leiters, also vor dem Entladungsabschnitt austritt und dass der Entladungsabschnitt keine exakt definierte Position zum Ende des Arbeitskanals aufweist, an welchem das Gas austritt. Andererseits ist es erheblich einfacher, den Leiter als isolierten Draht ohne ein Lumen auszubilden, was die Kosten der Herstellung senkt und die Herstellung von Einmal-Leitern ermöglicht. Diese wiederum verringern das Infektionsrisiko.

Leiter ohne Lumen sind aber nicht nur einfacher herzustellen, es ist vielmehr auch möglich, Koagulationsvorrichtungen, mit einem erheblich niedrigeren Durchmesser des Leiters herzustellen, was wiederum eine weitere Miniaturisierung der Gesamtanordnungen erlaubt. Gleichzeitig wird dennoch ein erheblich größerer Strömungsquerschnitt der Gaszuführungsleitung sichergestellt, was den Vorteil hat, daß eine wesentlich verbesserte Regelung der Gaszuführung in einfachster Weise sichergestellt werden kann. Man kann nämlich davon ausgehen, daß der Gasdruck am distalen Ende des Arbeitskanals sich nur sehr unwesentlich vom Gasdruck am proximalen Ende unterscheidet, da bei den erzielbaren großen Strömungsquerschnitten nur ein geringer Druckabfall (bei üblichen, nicht allzu großen Strömungsgeschwindikeiten) zu erwarten ist. Damit kann gleichzeitig (bei einer entsprechenden Regelung des Gaszuführungsdrucks) der Gefahr vorgebeugt werden, bei einem gestörten Gasabfluß eine unkontrollierte, schädigende Insufflation zu verursachen.

Mit absoluter Sicherheit wird vermieden, daß ein zu starker, laminarer Gasstrom auf den Bereich des zu koagulierenden Gewebes auftrifft, was, wie erwähnt, eine Embolie verursachen könnte.

Vorzugsweise umfaßt der Leiter mindestens einen Draht, der mittels einer dicht aufgebrachten Isolatorschicht insbesondere gegenüber dem Endoskop bzw. gegenüber der Wand des - Arbeitskanals isoliert ist. Es handelt sich also hier um eine besonders einfach herzustellende Anordnung. Vorzugsweise wird in diesem Fall der Draht derart steif-elastisch ausgebildet, daß eine proximale Fixierung des Leiters eine hinreichende Fixierung auch seines distalen Endes und somit des Entladungsabschnittes sicherstellt. Es ist also nicht notwendig, den Leiter exakt koaxial zum Arbeitskanal zu führen und insbesondere in dessen Endbereich zu halten, da - überraschenderweise - der vorzugsweise sanft ausströmende Edelgas-Strom sozusagen eine "Wolke" bildet, welche den Bereich zwischen dem Entladungsabschnitt und dem zu koagulierenden Gewebe füllt.

Die Isolatorschicht ist vorzugsweise derartig ausgebildet, daß eine definierte, vorzugsweise der Frequenz des Koagulationsstroms angepaßte Kapazität zwischen Leiter und Wand entsteht, so daß eine optimale Leistungsanpassung sichergestellt werden kann. Insbesondere soll die Kapazität (Streukapazität) zwischen Leiter und Wand sehr niedrig gehalten werden, um die Verluste zu minimieren.

Vorzugsweise besteht die Isolatorschicht aus temperaturbeständigem Material, insbesondere aus Polytetrafluorethylen oder einem derartigen hochtemperaturbeständigem Kunststoff.

Die Schutzeinrichtung wird vorzugsweise derart ausgebildet, daß eine im wesentlichen ungerichtete Entladung am Entladungsabschnitt gewährleistet ist. Es soll also damit sichergestellt werden, daß der Entladungsstrom zwischen dem Entladungsabschnitt und einem solchen Gewebeabschnitt fließt, der eine relativ hohe Feuchtigkeit und damit einen relativ niedrigen Widerstand (pro Flächeneinheit) aufweist. Dadurch wird sichergestellt, daß die Koagulationsstrom-Entladungsstrecke sich ihren "Weg" selbsttätig such. Dadurch kann eine besonders gleichmäßige und schnelle Koagulationswirkung sichergestellt werden.

Es gibt nun verschiedene Möglichkeiten, die Schutzeinrichtung derart auszubilden, daß keine übergroßen, möglicherweise schädigenden Koagulationsströme fließen.

Bei einer ersten Ausführungsform der Erfindung ist die Schutzeinrichtung direkt aus der Isolatorschicht gebildet, indem diese an ihren Enden über die stromleitenden Teile (in Richtung des Leiters).hinausragend angeordnet sind. Hier ist eine Vielzahl von geometrischen Formen denkbar.

Alternativ kann die Schutzeinrichtung als gesondertes Teil, insbesondere als hülsen-, kugel- oder korbförmiges Teil aus isolierendem, temperaturbeständigem Material ausgebildet sein. Besonders eignet sich hierfür Keramikmaterial.

Vorzugsweise umfaßt der Entladungsabschnitt im wesentlichen punkt- oder spitzenförmige Entladungselektroden. Durch diese Maßnahme ist eine leichte Zündung des Plasmas durch die an Spitzen entstehenden hohen Feldstärken gewährleistet. Diese Maßnahme erhöht also die Arbeitssicherheit, da keine allzu hohen Spannungen zum Zünden der Entladung verwendet werden müssen.

Bei einer besonders bevorzugten Ausführungsform der Erfindung umfaßt der Entladungsabschnitt nun eine Vielzahl von Entladungselektroden, die elektrisch parallelgeschaltet und im wesentlichen eine stetige Fläche definierend, im wesentlicher. äquidistant durch Isolator-Schichten getrennt, angeordnet sind. Bei Verwendung derartig gleichmäßig verteilter, punktförmiger Elektroden, die darüber hinaus im wesentlichen vorzugsweise oberflächenbündig zur Isolatorschicht in einer Ebene oder in einer konvexen Fläche angeordnet sind, ist nun selbst bei direktem Kontakt mit den Elektroden und dem Gewebe sichergestellt, daß keine ernsthafte Gewebeschädigung auftritt. Bei einer derartigen Anordnung bildet sich nämlich im Bereich der Kontaktfläche einer jeden punktförmigen Elektrode eine im wesentlichen halbkugelförmige Stromverteilung aus (es besteht ja lediglich Kontakt an der Oberfläche des Gewebes und die Elektrode kann nicht in das Gewebe eindringen), was wiederum dazu führt, daß die Stromdichte bereits in sehr kurzer Entfernung zur Elektrode so niedrige Werte annimmt, daß keine ernsthafte Gewebeschädigung mehr auftritt. Im direkten Kontaktbereich wiederum, in welchem die Stromdichte noch relativ hoch ist und die im Gewebe enthaltene Flüssigkeit verdampft, steigt der Widerstand sehr schnell an, so daß der Stromfluß insgesamt unterbunden wird. Dadurch wiederum, daß eine Vielzahl von Einzelelektroden vorgesehen ist, kann sichergestellt werden, daß ein gleichmäßiger, über einen relativ großen Flächenbereich strömender Plasma-Koagulationsstrom fließt.

Vorzugsweise sind Fixier- und/oder Justiereinrichtungen insbesondere am proximalen Ende des Endoskops vorgesehen und derart ausgebildet, daß der Entladungsabschnitt im Blickfeld der Beobachtungsoptik des Endoskops feststellbar ist.

Bei einer anderen bevorzugten Ausführungsform der Erfindung sind die Fixier- und Justiereinrichtungen derart ausgebildet, wie dies für Instrumente üblich ist, die in den Arbeitskanal von Endoskopen einführbar und aus dessen distalen Ende heraussuchend bewegbar bzw. manipulierbar sind. Die eigentliche Manipuliervorrichtung sitzt hierbei am patientenfernen Ende des Endoskops und sind derart ausgebildet, daß das distale Ende des Leiters mit dem Entladungsabschnitt im Blickfeld des Endoskops bewegt werden kann. Auf diese Weise kann auch dann eine präzise Bearbeitung des Gewebes vorgenommen werden, wenn das Endoskop relativ zum Gewebe unbeweglich ist.

Nachfolgend werden bevorzugte Ausführungsformen der Erfindung anhand von Abbildungen näher erläutert. Hierbei zeigen:
- Fig. 1: eine schematisierte Teil-Schnittansicht eines Endoskops mit teilweise geöffnetem Arbeitskanal,
- Fig. 2: eine erste Ausführungsform eines Entladungsabschnitts mit Schutzvorrichtung im Längsschnitt,
- Fig. 3: eine zweite Ausführungsform eines Entladungsabschnitts mit Schutzvorrichtung im Längsschnitt,
- Fig. 4: eine dritte Ausführungsform eines Entladungsabschnitts mit Schutzvorrichtung im Längsschnitt,
- Fig. 5: eine vierte Ausführungsform eines Entladungsabschnitts mit einer Vielzahl von Elektroden im Längsschnitt,
- Fig. 6: eine Ansicht auf den Entladungsabschnitt nach Fig. 5 entlang der Linie VI-VI,
- Fig. 7: eine Ansicht auf eine fünfte Ausführungsform eines Entladungsabschnitts mit einer Vielzahl von Elektroden im Längsschnitt und
- Fig. 8: eine Seitenansicht des Entladungsabschnittes nach Fig. 7.

In der nachfolgenden Beschreibung werden für gleiche und gleichwirkende Teile dieselben Bezugsziffern verwendet..

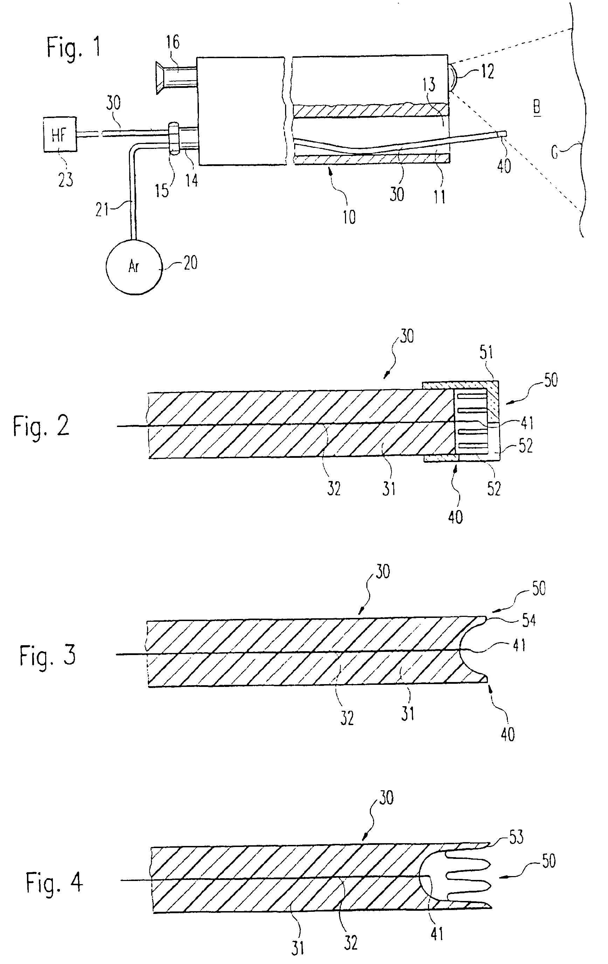

In Fig. 1 ist eine sehr stark schematisierte Seitenansicht eines Endoskops 10 gezeigt, das in an sich bekannter Weise einen Arbeitskanal 11 aufweisz, der sich von einem proximalen Arbeitskanalende 14 bis zu einem distalen Arbeitskanalende 13 erstreckt. Am distalen Ende des Endoskops 10 ist eine Optik 12 vorgesehen, die im Bereich eines Blickfeldes B befindliche Objekte, insbesondere zu koagulierendes Gewebe G, im allgemeinen auf einem Fernsehschirm wiedergibt. Im vorliegenden Fall wurde zur Verdeutlichung ein Okular 16 am proximalen Ende des Endoskops 10 gezeigt.

Am distalen Ende 14 des Arbeitskanals 11 befindet sich ein Y-Stück 15, durch welches einerseits eine Zuleitung 21 gasdicht angeschlossen ist, die zu einer Argonquelle 20 führt, welche (nicht gezeigte) Regeleinrichtungen aufweist, um einen voreinstellbaren, gleichmäßigen Strom von Argongas in den Arbeitskanal 11 an dessen proximalem Ende 14 einströmen zu lassen, so daß das Argon am distalen Arbeitskanalende 13 gleichmäßig mit geringer Strömungsgeschwindigkeit als "turbulente Wolke" ausströmt und den Bereich zwischen einem zu koagulierenden Gewebe G und einem distalen Entladungsabschnitt 40 füllt, der das Ende eines Leiters 30 bildet. Der Leiter 30 wiederum führt durch den Arbeitskanal 11 und erstreckt sich (unter Abdichtung) durch das Y-Stück 15 zu einer HF-Quelle 23. Die HF-Quelle 23 ist in an sich bekannter Weise ausgebildet und erlaubt es, über den Leiter 30 dem Entladungsabschnitt 40 einen hochfrequenten Koagulationsstrem zuzuführen. Derartige HF-Generatoren sind auf dem Markt erhältlich.

Nachfolgend werden besondere Ausführungsformen des Entladungsabschnitts anhand der Fig. 2 bis 6 näher beschrieben.

Aus den Fig. 2 bis 4 geht hervor, daß der Leiter 30 einen Draht 32 umfaßt, der von einer Isolatorschicht 31 vorzugsweise aus PTFE umgeben ist. Der Draht 32 mit der ihn umgebenden Isolatorschicht 31 ist so steif, daß er - wie in Fig. 1 gezeigt - durch das fixierende Y-Stück 15 am proximalen Ende in einer Position gehalten wird, in welcher sein Entladungsabschnitt 40 in einer definierten, auch bei Bewegung des Endoskops 10 im wesentlichen gleichbleibenden Position innerhalb des Blickfeldes B der Optik 12 bleibt.

Bei der in Fig. 2 gezeigten Ausführungsform der Erfindung ist der Draht 32 über das Ende der Isolatorschicht 31 hinausragend angeordnet, so daß eine Entladungselektrode 41 gebildet ist. Vorzugsweise ist diese derart abgezwickt oder angespitzt, daß scharfe Kanten entstehen, an welchen die elektrische Feldstärke (wie an sich bekannt) sehr hoch wird, so daß ein Zünden des Plasmas schon bei relativ niedrigen Spannungen möglich ist.

Um nun zu verhindern, daß die Entladungselektrode 41 mit dem zu koagulierenden Gewebe G in einen direkten, stromleitenden Kontakt kommt, der zu einer ernsthaften und gefährlichen Gewebeschädigung (bei dünnen Gewebeschichten auch zu einem Durchbruch) führen könnte, ist als Schutzeinrichtung 50 ein Korb 51 vorgesehen, der in diesem Fall aus nicht leitendem, hochtemperaturbeständigem Keramikmaterial besteht. Der Korb 51 weist Öffnungen 52 auf, so daß ein direkter Gaskontakt zwischen der Außenumgebung und dem Inneren des Korbes möglich ist und sich ein im wesentlichen ungestörter Stromfluß durch ein Plasma ausbilden kann.

Bei den in den Fig. 3 und 4 gezeigten Ausführungsformen der Erfindung ist die Schutzeinrichtung 50 durch das Material der Isolatorschicht 31 gebildet. Bei der in Fig. 3 gezeigten Ausführungsform der Erfindung ist hierzu eine konkave Ausnehmung am Entladungsabschnitt 50 vorgesehen, an deren Grund die Entladungselektrode 41 sitzt und die einen Rand 54 aufweist, der in distaler Richtung über die Entladungselektrode 41 hervorsteht. Dadurch ist gewährleistet, daß ein direkter Kontakt zwischen der Entladungselektrode 41 und einem zu koagulierenden Gewebe G nicht stattfinden kann.

Bei der in Fig. 4 gezeigten Ausführungsform wird von einem ähnlichen Prinzip ausgegangen, wobei aber der Rand 54 nicht geschlossen sondern durch eine Reihe von Zähnen 53 gebildet ist, die ebenfalls einen direkten Kontakt der Entladungselektrode 41 mit dem zu koagulierenden Gewebe G verhindern.

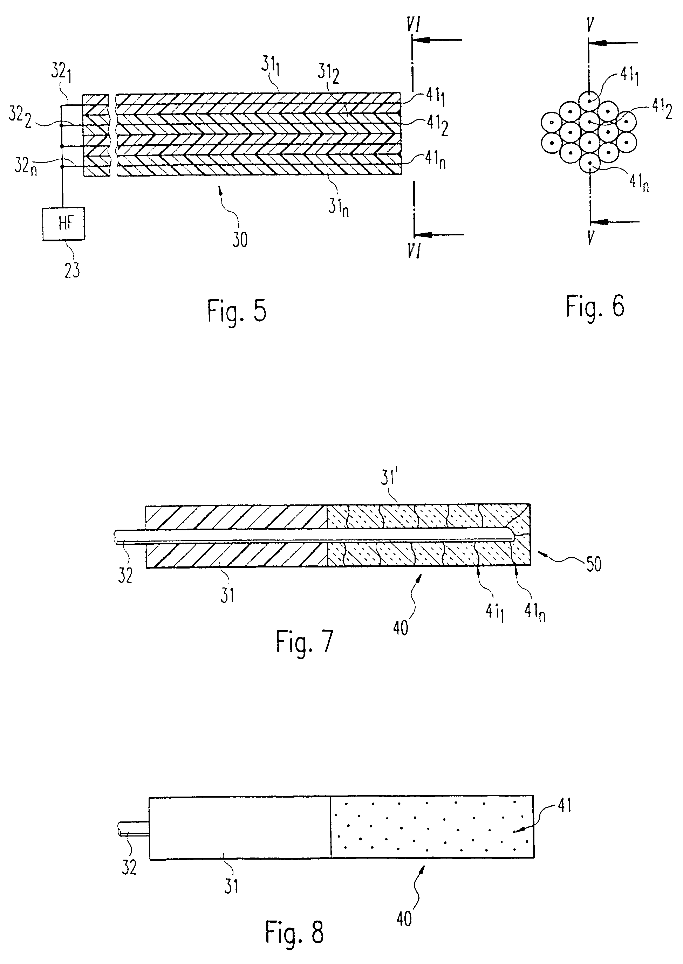

Die in den Fig. 5 und 6 gezeigte bevorzugte Ausführungsform der Erfindung basiert auf einem anderen Prinzip für die Schutzeinrichtung 50. Bei dieser Anordnung wird ein ganzes Bündel von Leitern 32₁, 32₂, ..., 32ₙ mit umgebenden Isolator-schichten 31₁, 31₂, ..., 31ₙ zusammengefaßt, wobei die Leiter 32 parallelgeschaltet und mit der HF-Quelle 23 verbunden sind. Die Leiter 30 sind am distalen Ende eine plane Fläche (bei einer anderen Ausführungsform eine konvexe Fläche) definierend abgeschnitten, so daß die Enden der Drähte 32 punktförmige Entladungselektroden 41₁, 41₂, ..., 41ₙ bilden, die bündig mit den Endflächen der Isolatorschichten 31 verlaufen. Kommt eine solche Endfläche, gebildet aus einer Isolatorschicht mit äquidistant darin verteilten punktförmigen Entladungselektroden 41 (wie in Fig. 6 gezeigt) mit einem Gewebe G in Verbindung, so ergibt sich ein Stromfluß, der sich von jeder der punktförmigen Kontaktstellen halbkugelförmig ausbreitet, wobei die Stromdichte mit dem Reziprokwert der dritten Potenz zum Abstand von der punktförmigen Elektrode abnimmt. Dadurch ist gewährleistet, daß bereits in sehr niedrigen Gewebetiefen eine "ungefährliche" Stromdichte herrscht.

Bei dem in Fig. 7 gezeigten Ausführungsbeispiel der Erfindung ist ebenfalls eine Vielzahl von punktförmigen Entladungselektroden 41₁, 41ₙ vorgesehen, die bündig mit der Endfläche einer Isolatorschicht 31' verlaufen. Die Isolatorschicht 31' ist in diesem Fall als Keramikteil ausgebildet, in welchem die Entladungselektroden in Form einer Füllung eingebettet sind. Dadurch wird gewährleistet, daß - wie in Fig. 8 gezeigt - eine relativ dicht mit Entladungselektroden 41 besetzte Fläche entsteht, so daß die Plasma-Entladung ungerichtet am Entladungsabschnitt 40 entstehen kann. Bei einer anderen, hier nicht gezeigten Ausführungsform der Erfindung ist der Entladungsabschnitt 40 aus einem (metallischen) Leiterstück gebildet, das mit einer dünnen Isolationsschicht überzogen ist, in der kleine Öffnungen oder Löcher zur Bildung der punktförmigen Entladungselektroden ausgebildet sind.

Aus obigem geht hervor, daß ein wesentlicher Punkt der vorliegenden Erfindung darin liegt, daß man nicht mehr - wie bisher üblich - einen "Schlauch" mit darin befindlicher Elektrode bzw. Stromzuführungseinrichtung verwendet und das Edelgas durch diesen Schlauch direkt in den Bereich "bläst", in welchem die Entladung stattfinden soll, sondern vielmehr den Arbeitskanal selbst verwendet und das Edelgas sozusagen unspezifisch (im Hinblick auf den Entladungsbereich) zuführt. Der Ort wiederum, von dem aus die Entladung "beginnt" ist genau definiert und sehr leicht so einstellbar, daß der operierende Arzt im wesentlichen ohne schwierige Manipulationen den Koagulationsvorgang im Griff hat.

## Patentansprüche

1. Koagulationsvorrichtung, umfassend
- ein Endoskop mit einem Arbeitskanal (11) mit einer proximalen und einer distalen Öffnung;
- eine Stromzuführungseinrichtung mit einem durch den Arbeitskanal (11) verlaufenden Leiter (30) zum Leiten eines Koagulationsstroms von einer HF-Quelle (23) zu einem Entladungsabschnitt (40) am distalen Ende des Leiters (30);
- eine Gaszuführung seinrichtung zum Zuführen von Edelgas von einer Edelgasquelle (20) durch eine Gasanssausströmöffnung zu einem Raum zwischen dem Entladungsabschnitt (40) und einem zu koagulierenden Gewebeabschnitt (G);
- eine Schutzeinrichtung (50) am Entladungsabschnitt (40) zum Verhindern eines übergroßen, schädigenden direkten Stromflusses durch Kontakt zwischen dem Entladungsabschnitt (40) und dem zu koagulierenden Gewebeabschnitt (G);
- wobei die Gaszuführungseinrichtung den als Leitung zum Transport des Edelgases benutzten Endoskop-Arbeitskanal (11) umfasst,
**dadurch gekennzeichnet, dass**
der Leiter (30) mit seinem distalen Ende und seinem Entladungsabschnitt (40) aus der distalen Öffnung (13) des Arbeitskanals (11), welche die Gasansströmöffnung bildet, derart frei hervor-ragt, dass aus dieser Öffnung (13), ausströmendes Edelgas vor dem Entladungsabschnitt (40) aus dem Endoskop-Arbeitskanal (11) austritt und das distale Ende des Leiters (30) und den Entladungsabschnitt (40) umspülend in den Raum zwischen dem Entladungsabschnitt (40) und dem zu koagulierenden Gewebeabschnitt (G) strömt.

2. Koagulationsvorrichtung nach Anspruch 1,
**dadurch gekennzeichnet, daß**
der Leiter (30) mindestens einen Draht (32) umfaßt, der mittels einer dicht aufgebrachten Isolatorschicht (31) insbesondere gegenüber dem Endoskop (10) bzw. der Wand des Arbeitskanals (11) isoliert ist.

3. Koagulationsvorrichtung nach Anspruch 2,
**dadurch gekennzeichnet, daß**
der mindestens eine Draht (32) derart steif-elastisch ausgebildet ist, daß eine proximale Fixierung des Leiters (30) eine hinreichende Fixierung des Entladungsabschnitts (40) sicherstellt.

4. Koagulationsvorrichtung nach einem der Ansprüche 2 oder 3,
**dadurch gekennzeichnet, daß** die Isolatorschicht (31) derart dick ausgebildet ist, daß eine definierte, vorzugsweise der Frequenz des Koagulationsstroms angepaßte Kapazität zwischen Leiter und Wand entsteht.

5. Koagulationsvorrichtung nach einem der Ansprüche 2 bis 4,
**dadurch gekennzeichnet, daß** die Isolatorschicht aus temperaturbeständigem Material, insbesondere aus polytetrafluorethylen ausgebildet ist.

6. Koagulationsvorrichtung nach einem der vorhergehenden Ansprüche,
**dadurch gekennzeichnet, daß** die Schutzeinrichtung (50) derart ausgebildet ist, daß eine im wesentlichen ungerichtete Entladung am Entladungsabschnitt (40) gewährleistet ist.

7. Koagulationsvorrichtung nach einem der Ansprüche 2 bis 6,
**dadurch gekennzeichnet, daß** die Schutzeinrichtung (50) aus der Isolatorschicht (31) gebildet ist.

8. Koagulationsvorrichrung nach einem der vorhergehenden Ansprüche,
**dadurch gekennzeichnet, daß** die Schutzeinrichtung (50) als gesondertes hülsen-, kugel- oder korbförmiges Teil (51, 52) aus isolierendem, temperaturbeständigem Material, insbesondere aus Keramik gebildet ist.

9. Koagulationsvcrrichtung nach einem der vorhergehenden Ansprüche,
**dadurch gekennzeichnet, daß** der Entladungsabschnitt (40) im wesentlich punkt- oder spitzenförmige Entladungselektroden (41) umfaßt.

10. Koagulationsvorrichtung nach Anspruch 9,
**dadurch gekennzeichnet, daß** eine Vielzahl von Entladungselektroden (41) vorgesehen ist, die elektrisch parallelgeschaltet und im wesentlichen eine stetige Fläche definierend angeordnet sind.

11. Koagulationsvorrichtung nach Anspruch 10,
**dadurch gekennzeichnet, daß** die Entladungselektroden (41) in einer Ebene angeordnet sind.

12. Koagulationsvorrichtung nach Anspruch 10,
**dadurch gekennzeichnet, daß** die Entladungselektroden (41) in einer konvexen Fläche angeordnet sind.

13. Koagulationsvorrichtung nach einem der Ansprüche 9 bis 12,
**dadurch gekennzeichnet, daß** die Entladungselektroden (41) im wesentlichen bündig mit einer Isolatorschicht (31) angeordnet sind.

14. Koagulationsvorrichtung nach einem der Ansprüche 9 bis 13,
**dadurch gekennzeichnet, daß** die Entladungselektroden (41) eine Vielzahl oder ein Bündel von isolierten Einzeldrähten (32) umfaßt, die jeweils endseitig abgezwickt oder plan abgeschnitten sind.

15. Koagulationsvorrichtung nach Anspruch 1,
**gekennzeichnet durch**
eine Manipulationsvorrichtung, die derart ausgebildet und mit dem Leiter (30) in Wirkverbindung angeordnet ist, daß das distale Ende des Leiters (30) bzw. sein Entladungsabschnitt (40) insbesondere bei relativ zum Gewebeabschnitt (G) feststehenden Endoskop nach Art eines Endoskop-Arbeitsinstrumentes bewegbar ist.

## Claims

1. A coagulator comprising
- an endoscope having a working channel (11) with a proximal and a distal opening;
- a current supply means having a conductor (30) extending through the working channel (11) for conducting a coagulation current from an HF source (23) to a discharge portion (40) at the distal end of the conductor (30);
- a gas supply means for supplying inert gas from an inert gas source (20) through a gas outflow opening to a space between the discharge portion (40) and a tissue portion (G) to be coagulated;
- a protective device (50) at the discharge portion (40) for preventing an excessive, damaging, direct current flow by contact between the discharge portion (40) and the tissue portion (G) to be coagulated;
- the gas supply means comprising the endoscope working channel (11) used as conductor for conveying the inert gas,
**characterised in that**
the conductor (30) projects freely with its distal end and its discharge portion (40) out of the distal opening (13) of the working channel (11), which forms the gas outflow opening, in such a way that inert gas flowing out of this opening (13) exits from the endoscope working channel (11) upstream of the discharge portion (40) and flows round the distal end of the conductor (30) and the discharge portion (40) into the space between the discharge portion (40) and the tissue portion (G) to be coagulated.

2. A coagulator according to claim 1,
**characterised in that**
the conductor (30) comprises at least one wire (32), which is insulated in particular relative to the endoscope (10) or the wall of the working channel (11) by means of a tightly applied insulator layer (31).

3. A coagulator according to claim 2,
**characterised in that**
the at least one wire (32) is of rigid resilient construction, such that proximal fixation of the conductor (30) ensures adequate fixation of the discharge portion (40).

4. A coagulator according to one of claims 2 or 3,
**characterised in that**
the insulator layer (31) is of such thickness that a defined capacitance arises between conductor and wall, preferably adapted to the frequency of the coagulation current.

5. A coagulator according to any one of claims 2 to 4,
**characterised in that**
the insulator layer is made of heat-resistant material, in particular of polytetrafluoroethylene.

6. A coagulator according to any one of the preceding claims,
**characterised in that**
the protective device (50) is so constructed that substantially undirected discharge is ensured at the discharge portion (40).

7. A coagulator according to any one of claims 2 to 6,
**characterised in that**
the protective device (50) is formed of the insulator layer (31).

8. A coagulator according to any one of the preceding claims,
**characterised in that**
the protective device (50) takes the form of a separate sleeve-shaped, spherical or cage-shaped part (51, 52) of insulating, heat-resistant material, in particular of ceramics.

9. A coagulator according to any one of the preceding claims,
**characterised in that**
the discharge portion (40) comprises substantially punctiform or pointed discharge electrodes (41).

10. A coagulator according to claim 9,
**characterised in that**
a plurality of discharge electrodes (41) is provided, which are connected electrically in parallel and arranged substantially to define a continuous surface.

11. A coagulator according to claim 10,
**characterised in that**
the discharge electrodes (41) are arranged in a plane.

12. A coagulator according to claim 10,
**characterised in that**
the discharge electrodes (41) are arranged over a convex surface.

13. A coagulator according to any one of claims 9 to 12,
**characterised in that**
the discharge electrodes (41) are arranged substantially flush with an insulator layer (31).

14. A coagulator according to any one of claims 9 to 13,
**characterised in that**
the discharge electrodes (41) comprise a plurality or a bundle of insulated individual wires (32), which are each pinched off or cut off flat at the ends.

15. A coagulator according to claim 1,
**characterised by**
a manipulation device, which is constructed in such a way and arranged in active connection with the conductor (30) that the distal end of the conductor (30) or its discharge portion (40) may be moved in the manner of an endoscopic instrument in particular in the case of an endoscope which is stationary relative to the tissue portion (G).

## Revendications

1. Dispositif de coagulation, comprenant :
- un endoscope comportant un canal de travail (11) ayant une ouverture proximale et une ouverture distale ;
- un dispositif d'amenée de courant comportant un conducteur (30) s'étendant à travers le canal de travail (11) pour faire passer un courant de coagulation d'une source HF (23) à une section de décharge (40) située à l'extrémité distale du conducteur (30) ;
- un dispositif d'amenée de gaz pour amener un gaz rare d'une source de gaz rare (20), en passant par une ouverture de sortie de gaz, à un espace entre la section de décharge (40) et une section de tissu (G) devant être coagulée ;
- un dispositif de protection (50) à la section de décharge (40) pour empêcher un écoulement direct de courant excessif nocif par contact entre la section de décharge (40) et la section de tissu (G) devant être coagulée ;
- le dispositif d'amenée de gaz comprenant le canal de travail (11) de l'endoscope, utilisé en tant que conduite pour le transport du gaz rare,
**caractérisé en ce que**
le conducteur (30), par son extrémité distale et sa section de décharge (40), dépasse librement de l'ouverture distale (13) du canal de travail (11), qui forme l'ouverture de sortie de gaz, de telle sorte que le gaz rare sortant par cette ouverture (13) sort du canal de travail (11) de l'endoscope en avant de la section de décharge (40) et, en s'écoulant autour de l'extrémité distale du conducteur (30) et autour de la section de décharge (40), s'écoule dans l'espace entre la section de décharge (40) et la section de tissu (G) devant être coagulée.

2. Dispositif de coagulation selon la revendication 1,
**caractérisé en ce que** le conducteur (30) comprend au moins un fil (32), qui est isolé en particulier vis-à-vis de l'endoscope (10) ou de la paroi du canal de travail (11) à l'aide d'une couche isolante (31) appliquée serrée.

3. Dispositif de coagulation selon la revendication 2,
**caractérisé en ce qu'**au moins un fil (32) a un caractère rigide-élastique tel qu'une fixation proximale du conducteur (30) garantit une fixation suffisante de la section de décharge (40).

4. Dispositif de coagulation selon l'une des revendications 2 ou 3,
**caractérisé en ce que** la couche isolante (31) est réalisée suffisamment épaisse pour qu'il se crée entre le conducteur et la paroi une capacité définie, de préférence adaptée à la fréquence du courant de coagulation.

5. Dispositif de coagulation selon l'une des revendications 2 à 4,
**caractérisé en ce que** la couche isolante est réalisée en un matériau résistant en température, en particulier en polytétrafluoréthylène.

6. Dispositif de coagulation selon l'une des revendications précédentes,
**caractérisé en ce que** le dispositif de protection (50) est réalisé de telle sorte qu'une décharge sensiblement non dirigée est assurée à la section de décharge (40).

7. Dispositif de coagulation selon l'une des revendications 2 à 6,
**caractérisé en ce que** le dispositif de protection (50) est constitué de la couche isolante (31).

8. Dispositif de coagulation selon l'une des revendications précédentes,
**caractérisé en ce que** le dispositif de protection (50) est réalisé sous forme d'une pièce (51, 52) distincte, en forme de douille, de sphère ou de panier, en un matériau isolant résistant en température, en particulier en céramique.

9. Dispositif de coagulation selon l'une des revendications précédentes,
**caractérisé en ce que** la section de décharge (40) comprend des électrodes de décharge (41) sensiblement ponctuelles ou en forme de pointe.

10. Dispositif de coagulation selon la revendication 9,
**caractérisé en ce qu'**il est prévu une multiplicité d'électrodes de décharge (41), qui sont montées électriquement en parallèle et qui sont disposées en définissant sensiblement une surface continue.

11. Dispositif de coagulation selon la revendication 10,
**caractérisé en ce que** les électrodes de décharge (41) sont disposées dans un plan.

12. Dispositif de coagulation selon la revendication 10,
**caractérisé en ce que** les électrodes de décharge (41) sont disposées dans une surface convexe.

13. Dispositif de coagulation selon l'une des revendications 9 à 12,
**caractérisé en ce que** les électrodes de décharge (41) sont disposées sensiblement en affleurement d'une couche isolante (31).

14. Dispositif de coagulation selon l'une des revendications 9 à 13,
**caractérisé en ce que** les électrodes de décharge (41) comprennent une multiplicité ou un faisceau de fils individuels isolés (32) qui sont chacun à l'extrémité coupé à la pince ou sectionné selon un plan.

15. Dispositif de coagulation selon la revendication 1,
**caractérisé par** un dispositif de manipulation, qui est configuré et disposé en relation active avec le conducteur (30) de telle sorte que l'extrémité distale du conducteur (30) ou sa section de décharge (40) est mobile, en particulier dans le cas d'un endoscope fixé par rapport à la section de tissu (G) à la manière d'un instrument de travail de type endoscope.
